# EUROPEAN PATENT SPECIFICATION

(11) **EP 2 822 519 B1**
(45) Date of publication and mention of the grant of the patent: **06.04.2016**
(21) Application number: 12753070.7
(22) Date of filing: 22.08.2012
(51) Int. Cl.: A61F 9/008, A61B 3/14, A61B 3/10

(54) **CORNEAL TISSUE DETECTION AND MONITORING DEVICE**
VORRICHTUNG ZUR HORNHAUTGEWEBEERKENNUNG UND -ÜBERWACHUNG
DISPOSITIF DE DÉTECTION ET DE SURVEILLANCE DE TISSU CORNÉEN

(43) Date of publication of application: 14.01.2015
(73) Proprietor: WaveLight GmbH, 91058 Erlangen (DE)
(72) Inventor: KOENIG, Karsten, 661 19 Saarbruecken (DE); VOGLER, Klaus, 99444 Blankenhain (DE); WUELLNER, Christian, 91094 Bräunigshof (DE); DONITZKY, Christof, 90542 Eckental (DE)
(74) Representative: Frenkel, Matthias Alexander
(86) International application number: PCT/EP2012/003558
(87) International publication number: WO 2014/029407

(56) References cited:
- WO-A1-2008/002278
- WO-A2-2011/103357
- US-A1- 2005 063 041
- MENG HAN ET AL: "Second-harmonic imaging of cornea after intrastromal femtosecond laser ablation", JOURNAL OF BIOMEDICAL OPTICS, S P I E - INTERNATIONAL SOCIETY FOR OPTICAL ENGINEERING, US, vol. 9, no. 4, 1 July 2004 (2004-07-01), pages 760-766, XP007910902, ISSN: 1083-3668, DOI: 10.1117/1.1756919
- F. APTEL ET AL: "Multimodal Nonlinear Imaging of the Human Cornea", INVESTIGATIVE OPHTHALMOLOGY & VISUAL SCIENCE, vol. 51, no. 5, 1 May 2010 (2010-05-01), pages 2459-2465, XP055060724, ISSN: 0146-0404, DOI: 10.1167/iovs.09-4586
- MENG HAN ET AL: "Second harmonic generation imaging of collagen fibrils in cornea and sclera", OPTICS EXPRESS, vol. 13, no. 15, 1 January 2005 (2005-01-01), page 5791, XP055060726, ISSN: 1094-4087, DOI: 10.1364/OPEX.13.005791
- HSIN-YUAN TAN ET AL: "Characterizing the thermally induced structural changes to intact porcine eye, part 1: second harmonic generation imaging of cornea stroma", JOURNAL OF BIOMEDICAL OPTICS, vol. 10, no. 5, 1 January 2005 (2005-01-01), page 054019, XP055060728, ISSN: 1083-3668, DOI: 10.1117/1.2012987

## Description

In eye surgery, such as LASIK surgery, information may be gathered for use in the surgery. For example, the shape or thickness of the corneal tissue before surgery or the depth of cuts made during surgery may be measured. As another example, images of any scars within the corneal tissue due to previous surgeries may be taken.

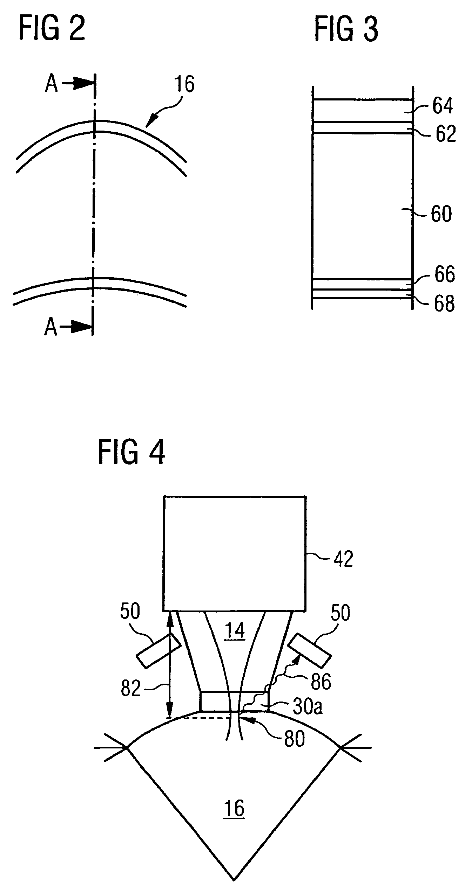

As shown in Fig. 3, the human cornea has five layers. The outer layer is the epithelium 64, a thin tissue layer of fast-growing and easily regenerated cells, typically composed of about six layers of cells. Next is the Bowman's layer 62, which is an 8-14µm thick condensed layer of collagen that protects the stroma. The stroma 60 is a thick, transparent middle layer that includes regularly arranged collagen fibers (also called "fibrils") and sparsely distributed interconnected keratocytes, which are cells responsible for general repair and maintenance. The Descemet's membrane 66 is an approximately 5-20µm thick acellular layer. Finally, the endothelium 68 is an approximately 5µm thick layer of mitochondria-rich cells.

The stroma 60 is the thickest layer of the cornea, accounting for up to 90% of the corneal thickness. The stroma is composed of about 200 plates of collagen fibrils called "lamellae", superimposed on one another. Each lamella is about 1.5-2.5 µm thick. The fibres of each lamella are parallel to one another, but generally at right angles to the fibers of adjacent lamellae. Fibres frequently interweave between adjacent layers. A remodelling, or reshaping, of the stroma during surgery alters the light-focussing capability of the cornea, which may correct a patient's vision.

A prevalent type of surgery for reshaping the cornea is LASIK (laser-assisted in situ keratomileusis) surgery, which is performed using a laser. LASIK surgery is typically performed in three steps. A first step creates a flap of corneal tissue. A second step remodels the cornea underneath the flap with a laser. In a third step, the flap is repositioned.

Before performing LASIK surgery, the thickness of the cornea is typically measured using at least one corneal pachymetry technique. During surgery, cutting of the corneal tissue as well as reshaping of the stroma layer may be monitored. It is typically desirable that the Descemet's membrane and endothelium remain unharmed while the flap is cut and the cornea is reshaped. Known diagnostic systems, however, have limited accuracy and image resolution and are difficult to integrate with the therapeutic systems used in LASIK surgery.

Known diagnostic devices for pre-operative and intra-operative diagnosis of corneal tissue include Scheimpflug cameras and Optical Coherence Tomography (OCT) scanners. Scheimpflug cameras used in corneal pachymetry have an image resolution limited to about 10µm. Scheimpflug cameras can be used to detect the position of the outer surfaces of the cornea, but do not provide information about the inner structure of the cornea.

Optical coherence tomography is an interferometric technique that is used to capture three-dimensional images from within optical scattering media, such as biological tissue. For applications in corneal pachymetry, OCT scanners have a resolution of about 5-10µm, which can be increased to approximately 1-2µm with known technologies. Wavelengths used for detection are typically in the range of 800-1300nm.

OCT scanners generate signals by detecting significant differences in the refractive indexes of adjacent tissues. The different refractive indexes of the adjacent tissues cause phase shifts in reflected, or back-scattered, light. However, tissue structures on a sub-micrometer scale and tissue boundaries that are not distinguished by a large difference in refractive index are not detectable. For example, the position and structure of the collagen fibrils of the stroma and the layered structure of the human cornea are not detectable using OCT scanning.

Scheimpflug cameras and OCT scanners use wavelengths that overlap the range of wavelengths used by surgical microscopes, e.g., in the visual range of 420 nm to 700nm. Therefore, using these detection devices intra-operatively can yield interference between the systems, leading to decreased measurement accuracy and/or compromised image quality.

Document Han et al., Second-harmonic Imaging of Cornea after Intra-stromal Femtosecond Laser Ablation, Journal of Biomedical Optics Vol. 9, No. 4, 760-766, relates to second-harmonic imaging of cornea after intra-stromal femtosecond laser ablation. For surgically treating a cornea sample, in particular, for intra-stromal ablation and flap cutting, a Neodym-glass (Nd:glass) laser providing light with a wavelength of 1060 nm and a pulse length of 700 fs is employed; the laser beam is guided to the eye through a lens pair with a variable focal length (Z-scan) and two galvanometer mirrors enabling a rapid XY-scan. The laser spot diameter in the focal plane is 5 µm. For second harmonic generation (SHG) imaging, a laser scanning multiphoton microscope is employed having a Titanium-Sapphire (Ti:Sa) laser, which is tunable from 700 to 1000 nm, as an excitation laser source with a laser emission wavelength set to 880 nm.

Document WO 2008/002278 A1 relates to optical imaging systems for analysis of biological tissue with a high optical scattering coefficient such as liver tissue. A nonlinear optical microscope apparatus images a sample by exciting SHG light in the sample in response to excitation light from an excitation source, which is a pulsed laser excitation source, wherein the power of the excitation light incident on the sample is such that it does not cause optical damage to the sample such as, for example, thermal denaturing. The SHG light emitted from the sample is then collected by a first optical condenser element and detected by a sensitive optical detector.

Document Aptel et al., Multimodal Nonlinear Imaging of the Human Cornea, Investigative Ophthalmology and Visual Science, Vol. 51, No. 5, 2459-2465, relates to multimodal nonlinear imaging of the human cornea. Imaging is performed on a laser scanning SHG and third harmonic generation (THG) microscope equipped with detection channels in backward and forward direction. Excitation is performed using a Ti:Sa oscillator and a synchronously pumped optical parametric oscillator delivering 100 to 150 fs pulses at the focus of an objective. The microscope incorporates galvanometer mirrors, motorized water-immersion objectives and photon-counting photomultiplier modules. For THG imaging, red-shifted excitation wavelengths of typically 1200 nm are used so that higher pulse energies can be used while preserving cell viability, wherein SHG imaging is performed with 860 nm excitation light.

Document US 2005/0063041 A1 relates to a microscopic imaging technique using both second and third harmonic waves of a excitation spectrum of a laser beam by a sample to form an image of the sample. A microscopic imaging system comprises a scanning device that receives a laser beam from a short pulse laser device. The laser beam, which is directed into the microscope by the scanning device, is focused by an objective lens of the microscope onto a biological sample. The laser beam has a predetermined wavelength, preferably of 1230 nm. Second and third harmonic wave components are directed to photo detectors and converted into corresponding electrical signals, which are then processed to generate an image of the sample. Document Han et al., Second Harmonic Generation Imaging of Collagen Fibrils in Cornea and Sclera, Optics Express, Vol. 13, No. 15, pages 5791-5797, relates to second harmonic generation imaging of collagen fibrils in cornea and sclera. The SHG imaging is performed on a laser scanning multi-photon microscope equipped with a mode-locked femtosecond Ti:Sa laser with a laser emission wavelength set to 800 nm. A water immersion objective is employed for focusing the excitation beam and for collecting of the backward SHG signals, which are directed to a photomultiplier tube detector.

Document Tan et al., Characterizing the Thermally Induced Structural Changes to Intact Porcine Eye, Part 1: Second Harmonic Generation Imaging of Cornea Stroma, Journal of Biomechanical Optics, Vol. 10, No. 5, pages 054019-1 to 054019-5, relates to second harmonic generation imaging of cornea stroma. The SHG microscope is a multiphoton microscope, wherein an 880 nm output of a diode-pumped solid state laser pumps a Ti:Sa laser, which is used as the SHG source. An average power of around 24 mW at the specimen is used for second harmonic signal generation and the SHG signal generated from the corneal collagen fibers is collected in backscattering geometry and detected by a single-photon collecting photomultiplier tube. Providing a separate diagnostic device, in addition to the therapeutic device, such as a femtosecond-pulse laser, increases the total cost of equipment needed to perform the surgery.

WO 2011/103357 discloses an optical coherence tomographic system for ophthalmic surgery. Amongst others, an imaging-guided system is shown, using one single pulsed laser to produce either surgical or imaging light.

It is therefore an object of examples of the invention to provide an eye-surgical laser apparatus that improves on existing apparatuses by enhancing measurement accuracy and/or use during surgery.

This object is achieved by an eye-surgical laser apparatus according to claim 1.

According to a first aspect of the invention, an eye-surgical laser apparatus in accordance with claim 1 is provided. The apparatus comprises optics that are adapted to focus a laser beam having a wavelength and a pulse length at a focus within a corneal tissue of an eye. Furthermore, the apparatus comprises a detection element adapted to detect, as an image-producing signal, light that is formed, at the focus, as a frequency multiple and backscattered to produce image information about the inner corneal tissue.

The detection of backscattered light at a frequency multiple allows high-resolution images to be captured, as the light at a frequency multiple has a shorter wavelength, and consequently provides an improved image resolution.

In one embodiment of the first aspect, the optics may be adapted to successively focus the laser beam at a further focus, which may be located at a depth different from the depth of the previous focus. The detection element is adapted to detect, as a further image-producing signal, light that is formed, at said further focus, as a frequency multiple and backscattered or forward emitted, to produce said image information about the inner corneal tissue. Collecting back-scattered light from two different focus depths enables the formation of a three-dimensional image by, e.g., computationally combining the image signals from both depths.

In a further embodiment of the first aspect, the optics may be adapted to successively focus the laser beam at a plurality of focuses at varying depths within the corneal tissue of the eye. The detection element can be adapted to detect, as image-producing signals, light that is formed, at each of said focuses, as a frequency multiple and backscattered or forward emitted, to compile three-dimensional image information about the inner corneal tissue. Collecting back-scattered light from a plurality of focus depths enables the formation of three-dimensional images by, e.g., computationally combining the image signals at the plurality of depths.

The use of the eye-surgical laser apparatus described above for scanning inner corneal tissue of an eye allows for the detection of backscattered light at a frequency multiple, which may be used to produce high-resolution images of corneal tissue while minimizing interference with the operation microscope.

The eye-surgical laser apparatus described above is usable for pre- or intra-operative diagnostic purposes. The wavelength of the laser beam is selected such that the beam energy in the focus of the laser beam is lower than an energy required for photodisruption of the corneal tissue.

The method of scanning a corneal tissue of an eye is not part of this invention. A laser beam having a wavelength and a pulse length is focused at a focus within a corneal tissue of an eye. A light that is formed, at the focus, as a frequency multiple and backscattered is detected as an image-producing signal, to produce image information about the inner corneal tissue.

The detection of backscattered light at a frequency multiple allows high-resolution images to be captured, as the light at a frequency multiple has a shorter wavelength, which typically provides an improved image resolution.

In this method, the laser beam may successively be focused at a further focus, which is located at a depth different from the depth of the previous focus. A light that is formed, at the further focus, as a frequency multiple and backscattered, is then detected as a further image-producing signal and may be used to produce image information about the inner corneal tissue. Collecting back-scattered light from two different focus depths enables the formation of a three-dimensional image by, e.g., computationally combining the image signals from both depths.

More generally, the laser beam may be successively focused at a plurality of focuses located at different depths within the corneal tissue of the eye, and the light that is formed, at each of the focuses, as a frequency multiple and backscattered can be detected as an image-producing signal, and compiled to produce three-dimensional image information about the inner corneal tissue. Collecting back-scattered light from a plurality of focus depths enables the formation of three-dimensional images by, e.g., computationally combining the image signals at the plurality of depths.

In any of the above aspects and embodiments, the plurality of focuses may be in the stroma of the eye, thus enabling a high-resolution detection and monitoring of layers and/or structures within the stroma.

In any of the above aspects and embodiments, the laser source is adapted to create a laser beam with variable wavelengths. In other words, the laser source is adapted to create a laser beam with variable pulse energy. As the pulse energy of the laser beam is dependent on its wavelength, in the context of this application, the terms "variable wavelength" and "variable pulse energy" may be used interchangeably. The provision of a variable wavelength / variable pulse energy allows the laser source to be used for both diagnostic and therapeutic purposes.

For diagnostic purposes, the laser source may be set to a wavelength and/or a pulse length corresponding to a low energy, e.g., the energy threshold is I≤10¹² W/cm², which will not cause photodisruption to the cornea, and image information is collected from backscattered light at a frequency multiple. For example, for diagnostic purposes, a wavelength of approximately 920 nm and a pulse length between 150-180 fs could be set; more generally, any wavelength and/or pulse length resulting in an energy which is lower than the energy threshold at which photodisruption of the stroma occurs is conceivable, e.g. the energy threshold may be I<10¹² W/cm².

For therapeutic purposes, the laser source may be set to a wavelength and/or pulse length corresponding to a sufficiently high energy to cause photodisruption of the cornea, e.g., the energy threshold is I≥10¹² W/cm². For example, for therapeutic purposes, a wavelength of approximately 1030 nm and a pulse length of approximately 300 or 350 fs could be set; more generally, any wavelength and/or pulse length resulting in an energy which is equal to or higher than the energy threshold at which photodisruption of the stroma occurs, e.g., the energy threshold may be I<10¹² W/cm².

In any of the above aspects and embodiments, the variable wavelength/pulse energy is varied depending on the depth of the focus within the corneal tissue of the eye. The wavelength may be variable between 700 and 1050 nm, or may be held at a particular wavelength such as 710 nm, 820 nm, 920 nm, or 1030 nm. Shorter wavelengths such as 700 nm produce higher-resolution image signals, while longer wavelengths such as 1050 nm can penetrate further into the corneal tissue. The succession of wavelengths of 710 nm, 820 nm, 920 nm, and 1030 nm allows a full scan of the cornea to be performed, while maintaining a high image resolution.

In any of the above aspects and embodiments, the pulse length of the laser beam can be a femtosecond pulse length between 10 and 400 fs, e.g., 100 fs, 350 fs. This pulse length provides sufficient energy for frequency multiples of the initial wavelength to be produced and backscattered toward the detection unit, yet ensures that the total energy of a laser pulse remains below the photodisruption threshold of the corneal tissue of the eye.

In any of the above aspects and embodiments, the pulse energy of the laser beam may be set such that the beam energy in the focus of the laser beam is at an energy level lower than the threshold for photodisruption of the corneal tissue, such that the apparatus is usable for pre- or inter-operative diagnostic purposes. Alternatively or in addition, when the apparatus is not being used for diagnostic purposes during surgery, the pulse energy of the laser beam may be set such that the beam energy in the focus of the laser beam is at an energy level which is equal to or exceeds the threshold for photodisruption of the corneal tissue, such that the apparatus is usable for therapeutic purposes.

In any of the above aspects and embodiments, the frequency multiple may be a Second-Harmonic Generation (SHG) or Third-Harmonic Generation (THG) signal.

The invention is defined in the claims and will be explained further on the basis of the appended figures, which are schematic throughout.
- Fig. 1a: shows a schematic block representation of elements of a laser system for eye-surgical treatments.
- Fig. 1b: shows a schematic block representation of elements of a laser system according to a variant of the laser system shown in Fig. 1a.
- Fig. 2: shows a schematic diagram of the cornea of a human eye.
- Fig. 3: shows the layers of corneal tissue along the cross-section A-A' of the eye shown in Fig. 2.
- Fig. 4: shows a block diagram illustrating the components of the laser apparatus shown in Fig. 1a.

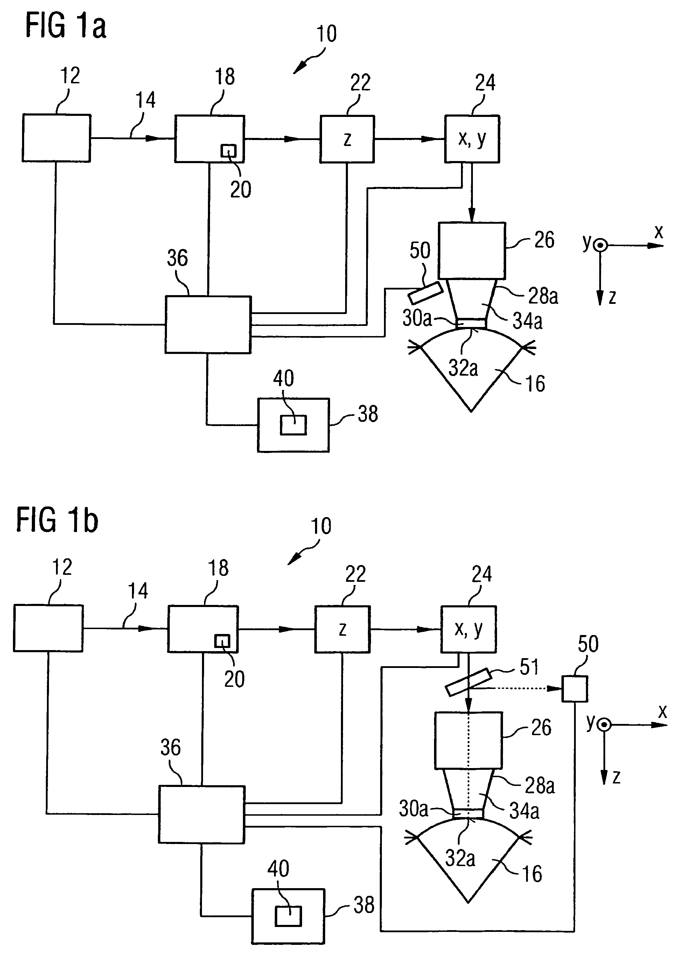

Figs. 1a and 1b show a laser system 10 comprising a laser apparatus for focusing a laser beam 14 at a focus point within an eye 16.

The laser system comprises a laser source 12. The laser source 12 may include, for example, a laser oscillator (e.g., solid-state laser oscillator); a pre-amplifier, which increases the pulse power of the laser pulses emitted from the oscillator and simultaneously temporally stretches them; a subsequent pulse-picker, which selects individual laser pulses from the pre-amplified laser pulses of the oscillator in order to lower the repetition rate to a desired degree; a power amplifier, which amplifies the selected, still temporally stretched, pulses to the pulse energy needed for the application; and a pulse compressor, which temporally compresses the pulses output from the power amplifier to the pulse duration desired for the application.

The laser source 12 generates a pulsed laser beam 14. The pulse duration of the radiation pulses is chosen either to generate reflected or backscattered light signals for diagnostic purposes or to create incisions in the corneal tissue of an eye 16 of a patient for treatment purposes. The radiation pulses of the laser beam 14 have a pulse duration in the nanosecond, picosecond, femtosecond or attosecond range.

The laser beam 14 generated by the laser source 12 furthermore has a pulse repetition rate suitable for the particular application. The repetition rate of the radiation pulses emitted from the laser device 10 and directed onto the eye 16 may correspond to the repetition rate of the radiation pulses that are generated at the output of the laser source 12. Alternatively, a portion of the radiation pulses emitted from the laser source 12 may be blanked by means of an optical switch 18 arranged in the radiation path of the laser beam 14 such that they do not reach the eye 16. This may be required by, e.g., a predetermined machining profile for the eye 16.

The optical switch 18, which is also called a pulse modulator, may be, for example, an acousto-optical modulator or an electro-optical modulator. Generally, the optical switch 18 may include arbitrary optically active elements that enable a rapid blanking of individual laser pulses. The optical switch 18 may include, for example, a beam trap, indicated schematically at 20, which serves to absorb radiation pulses to be blanked. The optical switch 18 can deflect such radiation pulses to be blanked from the normal beam path of the radiation pulses of the laser beam 14 and direct them onto the beam trap 20.

Further optical components that are arranged in the beam path of the laser beam 14 include a z-controller 22 and an x-y controller 24. The z-controller 22, on the one hand, controls the longitudinal location of the focal point of the laser beam 14; the x-y controller 24, on the other hand, controls the transverse location of the focal point. A coordinate frame that represents the x-y-z directions in the region of the eye 16 has been drawn in Figs. 1a and 1b for the purpose of illustration. In this context, the term 'longitudinal' refers to the direction of beam propagation, which conventionally is designated as the z-direction. Similarly, 'transverse' refers to a direction transverse to the direction of propagation of the laser beam 14, which conventionally is designated as the x-y plane.

To achieve a transverse deflection of the laser beam 14, the x-y controller 24 may include, for example, a pair of galvanometrically actuated scanner mirrors that are capable of tilting about mutually perpendicular axes. The z-controller 22 may include, for example, a longitudinally adjustable lens or a lens of variable refractive power or a deformable mirror with which the divergence of the laser beam 14, and consequently the z-position of the beam focus, can be controlled. Such an adjustable lens or mirror may be included in a beam expander that expands the laser beam 14 emitted from the laser source 12. The beam expander may, for example, be configured as a Galilean telescope.

The laser apparatus of the embodiments shown in Figs. 1a and 1b comprise a focusing objective 26 arranged in the beam path of the laser beam 14. The focusing objective 26 serve to focus the laser beam 14 onto a desired location on or in the eye 16, such as within the cornea. The focusing objective 26 is may be an f-theta focusing objective.

The optical switch 18, the z-controller 22, the x-y controller 24 and the focusing objective 26 do not have to be arranged in the order represented in Figs. 1a and 1b. For example, the optical switch 18 may, without loss of generality, be arranged in the beam path downstream of the z-controller 22. If desired, the x-y controller 24 and z-controller 22 may be combined to form a single structural unit. The order and grouping of the components shown in Figs. 1a and 1b is in no way to be understood as restrictive.

On the beam-exit side of the focusing objective 26, an applanation element 30a constitutes an abutment interface for the cornea of the eye 16. The applanation element 30a is transparent or translucent to the laser radiation. On the underside, facing towards the eye, the applanation element 30a includes an abutment face 32a for the cornea of the eye 16. In the exemplary case shown, the abutment face 32a is realised as a plane surface. In certain embodiments, the abutment face 32a is convex or concave. The abutment face 32a levels the cornea when the applanation element 30a is pressed against the eye 16 with appropriate pressure or when the cornea is aspirated onto the abutment face 32a by vacuum. As shown in Figs. 1a and 1b, the eye 16 is bearing against the planar abutment face 32a of the applanation element 30a.

The applanation element 30a, which in the case of plane-parallel design is ordinarily designated as the applanation plate, is fitted to the narrower end of a conically widening carrier sleeve 34a. The connection between the applanation element 30a and the carrier sleeve 34a may be permanent, for example by virtue of adhesion bonding, or it may be detachable, for instance, by virtue of a screw coupling. It is also conceivable to use a single optical injection-moulded part that functions as both the carrier sleeve 34a and the applanation element 30a. In a manner not represented in detail, the carrier sleeve 34a has coupling structures at its wider sleeve end, which in the drawing is the upper end. The coupling structures are suitable for coupling the carrier sleeve 34a onto the focusing objective 26.

The laser system 10 also comprises at least one detection element 50 that is adapted to detect light, which is formed as a frequency multiple at the focus and backscattered toward the detection element 50, in order to produce image information about the inner corneal tissue. The detection element 50 may be located either inside or outside the carrier sleeve 34a.

In the embodiment shown in Fig. 1b, a beam splitter 51, which may be a dichroic splitter, is provided in the beam path, and the detection element 50 is located in a position such that a portion of light deflected by the beam splitter 51 is deflected onto the detection element 50. In other words, the detection element 50 may be arranged such that the backscattered light, which is formed as a frequency multiple, is directly backscattered to the detection element 50 (Fig. 1a). Alternatively, the detection element 50 may be arranged such that the backscattered light is backscattered to the beam splitter 51 arranged in the beam path and is then deflected to the detection element 50 (Fig. 1b). The detection element 50 is a photodetector, for example, a photomultiplier tube (PMT), an Avalanche Photo Diode (APD), a high-gain Silicon Photomultiplier (SPM), or another type of amplifying light sensor.

The laser source 12, the optical switch 18, the detection element 50, and the two scanners 22, 24, are controlled by a control computer 36, which operates in accordance with a control program 40 stored in a memory 38. The control program 40 contains instructions (e.g., program code) that are executed by the control computer 36 so as to control the location of the beam focus of the laser beam 14 in the cornea, in the lens or at another location of the eye 16 bearing against the contact element 30a.

The laser system 10 may also comprise an interface module (not shown) connected to control computer 36 to allow a user to input commands to the control computer 36. The interface module may comprise a screen or monitor to enable the user to view status information about components of the laser system and/or data collected by at least one of the detection elements 50.

Figs. 2 and 3 schematically illustrate the cornea of a human eye. To illustrate the layers of the human cornea, the corneal layers of eye 16 are shown magnified in Fig. 3, as discussed in the introduction.

Fig. 4 schematically illustrates the components of the laser apparatus. As shown in Fig. 4, the laser apparatus comprises optics 42 that are adapted to focus a laser beam 14 within a corneal tissue of an eye 16. The optics 42 comprise at least the z-controller 22 and focusing objective 26 of the laser system 10 of Figs. 1a and 1b, but may also comprise the laser source 12, pulse-width modulator 18, and/or x-y controller 24 shown in Figs. 1a and 1b.

The laser apparatus also comprises at least one detection element 50, which is adapted to detect light that is formed as a frequency multiple at the focus and backscattered or forward emitted toward the detection element, to produce image information about the inner corneal tissue.

When the laser apparatus is used for therapeutic purposes, a beam 14 is generated with sufficient beam energy at the focus 80, which is located at a depth 82, so as to cut an incision pattern. In the course of a machining of the cornea, such an incision pattern completely severs a corneal tissue volume from the surrounding corneal tissue, as part of a corneal lenticule extraction or a corneal keratoplasty. If desired, this incision pattern may additionally subdivide the severed tissue volume into a plurality of volume segments individually separated from one another.

When the laser apparatus is used for diagnostic or scanning purposes, intense light at the focus 80 of the laser beam 14 causes highly polarized and noncentrosymmetric tissues, such as collagen, to produce light at a frequency multiple of the input frequency.

The higher-frequency light occurs partially in the form of Second Harmonic Generated (SHG) signals, which are created when two near-infrared photons interact with highly polarized, noncentrosymmetric materials to generate a single, visible photon with twice the energy and half the wavelength.

Higher-frequency light can also be produced in the form of Third Harmonic Generated (THG) signals, which are created when three near-infrared photons interact with highly polarized, noncentrosymmetric materials to generate a single, visible photon with three times the energy and one third the wavelength. While only the SHG and THG signals are described in detail here, it is noted that higher-order harmonic signals are also possible.

The light within the laser beam 14 causes collagen structures of the cornea that are located within the focus 80 to emit photons at frequency multiples of the frequency of the light which forms the laser beam 14. In one example, if the light within the laser beam 14 has a wavelength of λ =1030 nm, then a SHG signal is produced at the frequency λ_{SHG}=515 nm, and a THG signal is produced at λ_{THG}=343 nm.

When excited by the laser beam 14, higher-frequency light in the form of SHG and THG signals is emitted from the collagen structures of the cornea. The higher-frequency light is scattered in all directions, producing signals in the form of backscattered beams 86. The detection element 50 detects these signals and uses them to produce image information about the inner corneal tissue.

The diameter of the focus 80 of the laser beam 14 may be between approximately 1 µm and 10 µm. The diameter of the focus 80 of the laser beam 14 is selected to exceed the size of the structures or cells which are to be examined, e.g., the diameter of the focus 80 may be set to 1.5 µm.

The z-controller 22 of the laser system 10 is adapted to vary the depth 82 of the focus 80 within the eye 16. Furthermore, the laser source 12 of the laser system 10 is adapted to vary the wavelength of the light in the laser beam 14.

During diagnostic use, the wavelength of the light in the laser beam 14 can be varied between 700 and 1050 nm according to the depth of the focus 80 within the eye 16. Light with longer wavelengths travels more readily through the material of the eye 16, and therefore longer wavelengths can be used to examine material which is further removed from the outer surface of the eye 16.

Moreover, the pulse modulator 18 of the laser system 10 is adapted to vary the pulse energy of the laser beam 14. For example, the pulse energy of the laser beam 14 may be varied in the range of 0.5 µJ to 0.05 µJ.

In operation, a diagnostic scan may be performed by varying the pulse energy of the laser beam 14, such that the beam energy in the focus 80 of the laser beam 14 is lower than an energy required for photodisruption of the corneal tissue. The laser beam 14 is then focused at a focus 80 within the cornea of the eye 16, and the backscattered light 86 that is formed as a frequency multiple at the focus 80 is detected by the detection element 50 as an image-producing signal, to produce image information about the inner corneal tissue.

To compile three-dimensional image information, the laser beam 14 is successively focused at focusses 80 of varying depth 82 within the cornea. When performing diagnostic scans, the laser beam operates in a range of wavelengths between 700 and 1050 nm. At successive depths 82, the wavelength of the light which forms the laser beam 14 is increased, as the distance of the focus 80 from the outer surface of the eye 16 increases. Alternatively, a single wavelength within the range of 700-1050 nm may be used for multiple depths 82 or for all of the depths 82 within the eye 16.

After the diagnostic scan of the corneal tissue is complete, the pulse energy of the laser beam 14 is increased, such that the beam energy in the focus 80 of the laser beam 14 exceeds the energy required for photodisruption of the corneal tissue. At this point, surgery can begin or resume, and the laser system 10 is used for cutting and/or reshaping of corneal tissue.

In summary, the pulse energy of the laser beam 14 can be chosen such that the beam energy in the focus 80 of the laser beam 14 is above or below the photodisruptive energy of collagen. As such, the laser apparatus is alternately usable for either cutting/reshaping corneal tissue during surgery, or generating SHG/THG signals which are collected by the detection element 50 to produce diagnostic information about the cornea. In this way, a single laser system 10 can be alternately used to provide either diagnostic or therapeutic functionality.

## Claims

1. Eye-surgical laser apparatus, comprising
- a laser source (12) for creating a laser beam (14) with a variable wavelength,
- optics (26) that are adapted to focus a laser beam having a wavelength and a pulse length at a focus (80) within a corneal tissue (60-68) of an eye (16), and
- a detection element (50) adapted to detect, as an image-producing signal, light that is formed as a frequency multiple at the focus (80) and backscattered or forward emitted, wherein the light is detected to produce image information about the inner corneal tissue (60-68),
wherein
- the pulse energy of the laser beam (14) is either settable such that the beam energy in the focus (80) of the laser beam (14) is at an energy level equal to or exceeding the threshold for photodisruption of the corneal tissue (60-68), such that the laser apparatus is usable for therapeutic purposes, or
- the pulse energy of the laser beam is settable such that the beam energy in the focus (80) of the laser beam (14) is at an energy level lower than the threshold for photodisruption of the corneal tissue (60-68), such that the laser apparatus is usable for pre- or inter-operative diagnostic purposes, wherein the variable wavelength is varied depending on the depth of the focus (80) within the corneal tissue (60-68) of the eye (16).

2. Laser apparatus according to claim 1, wherein the optics (26) are adapted to successively focus the laser beam (14) at a plurality of focuses at varying depths within the corneal tissue (60-68) of the eye (16), wherein the detection element (50) is adapted to detect, as image-producing signals, light that is formed as a frequency multiple at each of said focuses (80) and backscattered or forward emitted, wherein the light is detected to gather three-dimensional image information about the inner corneal tissue (60, 62, 66).

3. Laser apparatus according to claim 2, wherein the plurality of focuses (80) are in the stroma (60) of the eye (16).

4. Laser apparatus according to one of the previous claims, wherein the wavelength is variable between 700 and 1050 nm.

5. Laser apparatus according to one of the previous claims, wherein a femtosecond pulse length is between 10 and 400 fs.

6. Laser apparatus according to one of the previous claims, wherein the frequency multiple is a Second-Harmonic Generated (SHG) or Third-Harmonic Generated (THG) signal.

## Patentansprüche

1. Augenchirurgische Laservorrichtung, umfassend
- eine Laserquelle (12) zum Erzeugen eines Laserstrahls (14) mit einer variablen Wellenlänge,
- Optiken (26), die ausgebildet sind, um einen Laserstrahl, der eine Wellenlänge und eine Pulslänge in einem Fokus (80) innerhalb des Hornhautgewebes (60-68) eines Auges (16) aufweist, zu fokussieren und
- ein Erfassungselement (50), das ausgebildet ist, um, als ein bilderzeugendes Signal, Licht zu erfassen, das als ein Frequenzvielfaches in dem Fokus (80) gebildet und zurückgestreut oder vorwärts emittiert wird, wobei das Licht erfasst wird, um Bildinformationen über das innere Hornhautgewebe (60-68) zu erzeugen, wobei
- die Pulsenergie des Laserstrahls (14) entweder derart einstellbar ist, dass die Strahlenergie in dem Fokus (80) des Laserstrahls (14) auf einem Energieniveau liegt, das gleich ist zu dem oder den Schwellwert für Photodisruption des Hornhautgewebes (60-68) derart übersteigt, dass die Laservorrichtung für therapeutische Zwecke anwendbar ist, oder
- die Pulsenergie des Laserstrahls derart einstellbar ist, dass die Strahlenergie in dem Fokus (80) des Laserstrahls (14) auf einem Energieniveau liegt, das niedriger als der Schwellenwert für Photodisruption des Hornhautgewebes (60-68) ist, derart, dass die Laservorrichtung für vor- oder intraoperative Diagnosezwecke anwendbar ist, wobei die variable Wellenlänge abhängig von der Fokustiefe (80) innerhalb des Hornhautgewebes (60-68) des Auges (16) variiert wird.

2. Laservorrichtung nach Anspruch 1, wobei die Optiken (26) ausgebildet sind, um den Laserstrahl (14) sukzessive auf eine Vielzahl von Fokussen bei variierenden Tiefen innerhalb des Hornhautgewebes (60 bis 68) des Auges (16) zu fokussieren, wobei das Erfassungselement (50) ausgebildet ist, als ein bilderzeugendes Signal, Licht zu erfassen, das als ein Frequenzvielfaches bei jedem der Fokusse (80) gebildet und zurückgestreut oder vorwärts emittiert wird, wobei das Licht erfasst wird, um dreidimensionale Bildinformationen über das innere Hornhautgewebe (60, 62, 66) zu sammeln.

3. Laservorrichtung nach Anspruch 2, wobei die Vielzahl von Fokussen (80) in dem Stroma (60) des Auges (16) liegt.

4. Laservorrichtung nach einem der vorangegangenen Ansprüche, wobei die Wellenlänge zwischen 700 und 1050 nm variiert.

5. Laservorrichtung nach einem der vorangegangenen Ansprüche, wobei eine Femtosekundenpulslänge zwischen 10 und 400 fs liegt.

6. Laservorrichtung nach einem der vorangegangenen Ansprüche, wobei das Frequenzvielfache ein Frequenzverdoppelungssignal oder Frequenzverdreifachungssignal ist.

## Revendications

1. Appareil laser pour chirurgie ophtalmologique, comprenant
- une source (12) de laser pour créer un faisceau laser (14) avec une longueur d'onde variable,
- une optique (26) qui est adaptée à focaliser un faisceau laser ayant une longueur d'onde et une longueur d'impulsion au niveau d'un foyer (80) à l'intérieur d'un tissu cornéen (60-68) d'un oeil (16), et
- un élément (50) de détection adapté à détecter, comme un signal de production d'image, une lumière qui est formée comme un multiple de fréquence au niveau du foyer (80) et rétrodiffusée ou émise directement, dans lequel la lumière est détectée pour produire des informations d'image relatives au tissu cornéen interne (60-68),
dans lequel
- l'énergie d'impulsion du faisceau laser (14) est réglable de telle sorte que l'énergie de faisceau dans le foyer (80) du faisceau laser (14) est à un niveau d'énergie égal ou supérieur au seuil de photodisruption du tissu cornéen (60-68), de telle sorte que l'appareil laser est utilisable à des fins thérapeutiques, ou
- l'énergie d'impulsion du faisceau laser (14) est réglable de telle sorte que l'énergie de faisceau dans le foyer (80) du faisceau laser (14) est à un niveau d'énergie inférieur au seuil de photodisruption du tissu cornéen (60-68), de telle sorte que l'appareil laser est utilisable à des fins de diagnostic pré- ou inter-opératoire, dans lequel la longueur d'onde variable est fait varier en fonction de la profondeur du foyer (80) à l'intérieur du tissu cornéen (60-68) de l'oeil (16).

2. Appareil laser selon la revendication 1, dans lequel l'optique (26) est adaptée à focaliser successivement le faisceau laser (14) en une pluralité de foyers à diverses profondeurs à l'intérieur du tissu cornéen (60-68) de l'oeil (16), dans lequel l'élément (50) de détection est adapté à détecter, comme des signaux de production d'image, une lumière qui est formée comme un multiple de fréquence au niveau de chacun desdits foyers (80) et rétrodiffusée ou émise directement, dans lequel la lumière est détectée pour réunir des informations d'image tridimensionnelle relatives au tissu cornéen interne (60, 62, 66).

3. Appareil laser selon la revendication 2, dans lequel la pluralité de foyers sont dans le stroma (60) de l'oeil (16).

4. Appareil laser selon l'une des revendications précédentes, dans lequel la longueur d'onde est variable entre 700 et 1 050 nm.

5. Appareil laser selon l'une des revendications précédentes, dans lequel une longueur d'impulsion femtoseconde est entre 10 et 400 fs.

6. Appareil laser selon l'une des revendications précédentes, dans lequel le multiple de fréquence est un signal généré comme deuxième harmonique (SHG) ou comme troisième harmonique (THG).
